# EUROPÄISCHE PATENTSCHRIFT

(11) **EP 2 576 062 B1**
(45) Veröffentlichungstag und Bekanntmachung des Hinweises auf die Patenterteilung: **22.04.2020**
(21) Anmeldenummer: 11727911.7
(22) Anmeldetag: 19.05.2011
(51) Int. Cl.: B01L 3/00, G01N 33/538

(54) **FLÜSSIGKEITSTRANSPORT- UND ANALYTISCHE TESTVORRICHTUNG**
DEVICE FOR TRANSPORTING AND ANALYSING FLUID
DISPOSITIF POUR LE TRANSPORT ET ANALYSIS D'UN FLUIDE

(30) Priorität: 07.06.2010 DE 102010022836
(43) Veröffentlichungstag der Anmeldung: 10.04.2013
(73) Patentinhaber: Sartorius Stedim Biotech GmbH, 37079 Göttingen (DE)
(72) Erfinder: PFLANZ, Karl, 37130 Gleichen (DE); JALLERAT, Eric, F-92410 Ville d'Avray (FR); HÄUSLER, Darius, 37154 Northeim (DE); HOLLAS, Markus, 38448 Wolfsburg (DE); ANDAG, Uwe, 37115 Duderstadt (DE)
(74) Vertreter: Schneider, Peter Christian
(86) Internationale Anmeldenummer: PCT/EP2011/002500
(87) Internationale Veröffentlichungsnummer: WO 2011/154090

(56) Entgegenhaltungen:
- EP-A1- 1 542 010
- EP-A1- 1 608 974
- WO-A2-01/02093
- US-A1- 2002 082 540
- US-B1- 6 420 622

## Beschreibung

### Gebiet der Erfindung

Die Erfindung bezieht sich auf eine analytische Testvorrichtung, umfassend eine Flüssigkeitstransportvorrichtung mit einem flüssigkeitsdichten Träger, darauf aufgebracht eine Startzone zur Aufbringung von zu transportierender Transportflüssigkeit und eine Zielzone, in die die Transportflüssigkeit zu transportieren ist, sowie eine sich zwischen der Startzone und der Zielzone erstreckende Leitungszone, die eine mikroporöse Transportschicht umfasst, in der die Transportflüssigkeit kapillarkraftvermittelt von der Startzone zur Zielzone strömt, wobei in wenigstens einer Zielzone ein selektiver Binder dauerhaft immobilisiert ist, der mit einem mit der Transportflüssigkeit aus der Startzone in die Zielzone transportierten, markierten Analyten selektiv bindungsfähig ist.

### Stand der Technik

Derartige analytische Testvorrichtungen sind bekannt aus der EP 1 608 974 B1.

Die US 2006/0205059 A1 offenbart - in einer speziellen Ausführungsform - eine allgemein als Lateral-Flow-Immunoassay bekannte analytische Testvorrichtung.

Der Lateral-Flow-Test basiert auf einem durch Kapillarkräfte getriebenen Flüssigkeitstransport in einer dünnen Schicht aus mikroporösem, offenporigem Material. Unter mikroporösem Material sei hier allgemein ein Material mit einer mittleren Porengröße von etwa 0,1 bis 50 Mikrometer und einem Volumenanteil der Poren von mindestens 30% verstanden. Typischerweise wird hierzu Zellulosenitrat verwendet, das in einer dünnen Schicht auf einem flüssigkeitsdichten Träger, z.B. einem Glas- oder Kunststoffträger, aufgebracht ist. Eine zu untersuchende Flüssigkeit, die hier als Probenflüssigkeit oder neutral als Transportflüssigkeit bezeichnet wird, wird an einer vorgesehenen Stelle der Testvorrichtung auf die mikroporöse Schicht aufgebracht. Dies kann z.B. durch Aufpipettieren der Transportflüssigkeit oder durch bereichsweises Eintauchen der Testvorrichtung in die Transportflüssigkeit oder auf andere Weise erfolgen. Häufig ist die Startzone zur Steigerung ihrer Flüssigkeitsaufnahmekapazität mit einem schwamm- oder vliesartigen Flüssigkeitsreservoir ergänzt, wobei im Rahmen der vorliegenden Erfindung lediglich von Bedeutung ist, dass die mikroporöse Transportschicht zu einem gegebenen Startzeitpunkt nur an einer oder mehreren definierten Stellen, nämlich der oder den Startzonen, mit der Transportflüssigkeit in Kontakt gebracht wird. Typischerweise ist die Startzone beim Lateral-Flow-Test mit einem selektiven Binder, der ein spezifischer Antikörper gegen einen mutmaßlich in der Transportflüssigkeit enthaltenen Analyten sein kann, derart belegt, dass der spezifische Binder im trockenen Zustand in der Startzone fixiert ist und im befeuchteten Zustand, d.h. nach Aufbringung der Transportflüssigkeit, beweglich ist und mit dieser strömen kann. Üblicherweise ist der selektive Binder mit einer detektierbaren Markierung, beispielsweise einem Fluoreszenz-Label, einem Gold- oder Latexpartikel, einer radioaktiven Markierung oder auf andere Weise markiert. Durch Bindung des selektiven Binders an tatsächlich in der Transportflüssigkeit enthaltenen Analyten wird letzterer somit markiert. Aufgrund der Kapillarkraft in der mikroporösen Transportschicht strömt die Transportflüssigkeit und mit ihr der markierte Analyt bzw. freie, selektive Binder entlang der Erstreckungsrichtung der Transportschicht auf eine vorbestimmte Zielzone zu. Die Ausgestaltung der Zielzone, einschließlich der Anzahl von Zielzonen hängt von Zweck und Ausgestaltung des jeweiligen Tests ab. Häufig wird eine Zielzone dadurch definiert, dass in ihr immobilisierte selektive Binder für den Analyten aufgebracht sind. Immobilisiert bedeutet im vorliegenden Zusammenhang, dass die selektiven Binder der Zielzone sowohl im trockenen als auch im befeuchteten Zustand ortsfest sind. Der bereits mit dem selektiven Binder der Startzone markierte Analyt wird an dem immobilisierten selektiven Binder der Zielzone gebunden und somit in der Zielzone fixiert. Man spricht hier von einer sogenannten Sandwich-Reaktion. Hierdurch kommt es zu einer Anreicherung der detektierbaren Markierung in der Zielzone. Eine solche Anreicherung erfolgt normalerweise nur, wenn in der Transportflüssigkeit tatsächlich Analyt vorhanden ist. Anderenfalls strömen die markierten, nicht an einen Analyten gebundenen selektiven Binder der Startzone durch die Zielzone hindurch.

Anreicherung der Markierung in der Zielzone bedeutet bei einer derartigen Testausgestaltung also das Vorhandensein von Analyt in der Transportflüssigkeit. Es sind jedoch auch andere Arten von Zielzonen bekannt. In sogenannten Kontrollzonen, die häufig stromabwärts der vorgenannten, ersten Art von Zielzonen liegen, können unspezifische Binder für den selektiven Binder der Startzone immobilisiert sein, sodass es in dieser zweiten Kontrollzone in jedem Falle einer erfolgreichen Testdurchführung zu einer Anreicherung von Markierung kommt. Auch können Zielzonen je nach Aufbau des Tests so gestaltet sein, dass ihre Anfärbung gerade auf ein Nicht-Vorhandensein von Analyt in der Transportflüssigkeit schließen lässt.

Bei solchen Lateral-Flow-Tests oder allgemeiner bei derartigen Flüssigkeitstransportvorrichtungen, wie sie üblichen Lateral-Flow-Tests zugrunde liegen, besteht ein bedeutendes Optimierungsdilemma. Einerseits ist ein möglichst schneller Flüssigkeitstransport von der Startzone zur Zielzone erwünscht. Dies ist insbesondere bei Tests im Home-Care-Bereich, in dem der Test von Laien durchgeführt wird, von wichtiger Bedeutung. Als Beispiel sei der bekannte Schwangerschaft-Streifentest genannt. Im Hinblick auf eine maximale Transportgeschwindigkeit wäre eine Transportschicht mit möglichst großer Porengröße günstig. Je größer nämlich die Porengröße ist, desto geringer ist der Strömungswiderstand und desto größer die resultierende Benetzungsgeschwindigkeit, d.h. die Transportgeschwindigkeit der Transportflüssigkeit in der Leitungszone. Daher werden bei üblichen Lateral-Flow-Tests als Transportschichten häufig Zellulosenitratschichten mit einer Porengröße von deutlich mehr als 3 Mikrometer verwendet. Andererseits ist im Hinblick auf eine Optimierung der Signalschärfe in der Zielzone eine möglichst kleine Porengröße günstig. Je kleiner nämlich die Porengröße, desto größer die innere Oberfläche der mikroporösen Schicht und je größer die innere Oberfläche, desto mehr selektive Binder sind in der Zielzone immobilisierbar, d.h. desto mehr Fänger für markierte Analyten stehen in der Zielzone zur Verfügung, was zu einem umso schärferen Signal führt. Schließlich ist bei diesem Optimierungsdilemma auch ein dritter Parameter zu berücksichtigen, nämlich die Schichtdicke der Transportschicht. Im Hinblick auf die Optimierung der Transportgeschwindigkeit wäre eine möglichst große Schichtdicke wünschenswert. Eine große Schichtdicke bedeutet jedoch auch ein großes Volumen der Transportschicht, welches, um einen Flüssigkeitsstrom zu realisieren, von der Transportflüssigkeit ausgefüllt werden muss. Bei vielen analytischen Tests steht jedoch nur eine geringe Menge an Flüssigkeit zur Verfügung, sodass es Verluste so gering wie möglich zu halten gilt. Unter diesem Aspekt wäre daher eine geringe Schichtdicke wünschenswert.

Die bekannten Flüssigkeitstransportvorrichtungen bzw. die darauf aufgebauten analytischen Testvorrichtungen lösen das geschilderte Optimierungsdilemma nicht wirklich. Vielmehr werden in allen Aspekten suboptimale Kompromisse realisiert, die je nach konkreter Bestimmung der Anwendung den einen oder den anderen der oben geschilderten Aspekte mehr oder weniger vernachlässigen.

Eine gattungsgemäße Vorrichtung ist bekannt aus der WO 2004/086042 A1. Dort ist ein Netzwerk aus mikroporösen Verbindungsgraten auf einem wasserundurchlässigen Träger aufgebracht. Hergestellt wird dieses Netzwerk, indem aus einer flächigen Schicht mikroporösen Materials bestimmte Bereiche (nämlich diejenigen, die im Endprodukt zwischen den Graten liegen sollen) mittels Durchtränkung mit wachsartigem Material blockiert oder mittels Laserablation gänzlich entfernt werden. Es resultiert ein auf die individuellen Grate verteilter und individualisierter Flüssigkeitsstrom, der eine Mehrzahl unterschiedlicher, unabhängiger Untersuchungen, die aus einer gemeinsamen Flüssigkeitsquelle gespeist werden, ermöglicht. Das o.g. Optimierungsproblem eines einheitlichen Lateral-Flow-Tests wird in der genannten Druckschrift weder angesprochen noch gelöst.

Die WO 03/025573 A1 offenbart einen weiteren Lateral-Flow-Test mit mehreren Start- und Zielzonen, wobei insbesondere letztere auch als eine großflächige Zielzone mit einer Mehrzahl von Teilzonen aufgefasst werden können. Im Übrigen birgt die Vorrichtung dieser Druckschrift ebenfalls die oben geschilderten Nachteile.

Aus der WO 2007/149043 A1 ist eine analytische Testvorrichtung bekannt, die auf mikroporöses Material als Transportschicht bzw. als Start- und Zielzone verzichtet. Vielmehr ist bei dieser Vorrichtung ein flüssigkeitsundurchlässiger Träger mit einer Vielzahl eng beieinander stehender, makroskopischer Höcker aus ebenfalls flüssigkeitsundurchlässigem Material versehen, die so eng beieinander stehen, dass sich ein kapillarkraftgetriebener Strom der Transportflüssigkeit von der Start- in die Zielzone ergibt. Nachteilig bei dieser Vorrichtung ist die geringe Möglichkeit der Fixierung einer ausreichenden Menge an Fängern in der Zielzone bzw. an markierten selektiven Bindern in der Startzone aufgrund der geringen Oberfläche, die zur Anhaftung zur Verfügung steht. Zudem ist die offene Struktur, die durch die Höcker gebildet wird, äußerst anfällig gegen Verdunstung, was im Hinblick auf einen möglichst sparsamen Umgang mit der Transportflüssigkeit als nachteilig angesehen werden muss.

Aus der DE 102 24 568 A1 ist eine miniaturisierte Microtiterplatte bekannt, die aus einem flüssigkeitsundurchlässigen Träger und darauf aufgebrachten, voneinander getrennten Höckern aus mikroporösem Material besteht. Die einzelnen Höcker können mit unterschiedlichen Bindern oder Chemikalien belegt werden. Eine zu untersuchende Flüssigkeit kann tröpfchenweise separat auf die Höcker aufgebracht und zur Reaktion mit dem Imprägnierungsbinder bzw. der Imprägnierungschemikalie gebracht werden. Auf diese Weise wird ein sogenanntes Micro-Array realisiert, welches die Beobachtung einer Vielzahl räumlich getrennt und zeitlich gleichzeitig ablaufender Reaktionen auf engstem Raum ermöglicht. Die einzelnen Schwämmchen sind voneinander durch Gräben getrennt, die einen Flüssigkeitsaustausch zwischen den einzelnen Höckern verhindern sollen.

Aus der DE 10 2005 014 691 A1 ist eine weitere Micro-Array-Vorrichtung bekannt, bei der sogenannte Wells, d.h. Töpfchen, eng benachbart in eine Schicht aus mikroporösem Material geschnitten sind. Die Töpfchen eignen sich zur Aufnahme lebender Zellen, das mikroporöse Wandmaterial, das jeweils die Töpfchen einer Gruppe miteinander verbindet, dient der gleichmäßigen Verteilung von Nährflüssigkeit auf die Töpfchen. Der Transport der Nährflüssigkeit innerhalb einer Töpfchengruppe folgt getrieben von der durch die Mikroporen des Wandmaterials aufgebauten Kapillarkraft. Die Zuführung der Nährlösung zu jeder Töpfchengruppe erfolgt durch herkömmliche Mikrofluidik-Kanäle, die in das flüssigkeitsundurchlässige Trägermaterial eingearbeitet sind. Am Transport der Flüssigkeit vom Aufbringungsort zum Zielort, d.h. zur jeweiligen Töpfchengruppe, ist daher bei der bekannten Vorrichtung keine Leitungszone beteiligt, die mikroporöses Material umfassen würde.

Ein ähnliches System offenbart die DE 10 2007 036 906 A1. Auch diese Druckschrift nimmt sich des Problems an, individuelle, unabhängige Strömungswege bereitzustellen. Hierzu werden zwei Lösungen angeboten. Zum einen wird auch hier die Idee des Verstopfens der Poren in Bereichen, in denen kein Flüssigkeitsstrom erwünscht ist, vorgestellt. Zum anderen werden Grate aus Verbundmaterial aus Mikrokugeln geschaffen, in denen der Flüssigkeitstransport stattfinden soll. Diese Grate werden entweder auf eine Trägeroberfläche aufgetragen oder in Rinnen in der Trägeroberfläche hineingerakelt. Das o.g. Optimierungsproblem eines einheitlichen Lateral-Flow-Tests wird in der genannten Druckschrift weder angesprochen noch gelöst.

Auch die US 2004/0022691 A1 geht das Problem der Schaffung individueller, unabhängiger Strömungswege an und löst es mit der Erzeugung unabhängiger Grate mikroporösen Materials zwischen zu verbindenden Vertiefungen (sog. wells). In einem Ausführungsbeispiel sind die Flüssigkeitstransport-Grate von noch höheren Wänden umgeben, sodass sie gesamt-topographisch als Rinnen erscheinen, funktional aber tatsächlich als Grate wirken. Das o.g. Optimierungsproblem eines einheitlichen Lateral-Flow-Tests wird in der genannten Druckschrift weder angesprochen noch gelöst.

Aus der US 6,420,622 B1 ist eine als Wundauflage und Wunddrainage geeignete Struktur zum Abtransport von Wundflüssigkeit bekannt. Die Struktur besteht aus parallelen Rinnen und Stegen mit einer Breite zwischen 50 und 3000 µm und einer Tiefe der Rinnen (bzw. Höhe der Stege) zwischen 5 und 3000 pm, wobei auch Poren eine beim Flüssigkeitstransport relevante Rolle spielen.

### Aufgabenstellung

Es ist die Aufgabe der vorliegenden Erfindung, eine gattungsgemäße Vorrichtung derart weiterzubilden, dass sie als Grundlage eines einheitlichen Lateral-Flow-Tests mit beschleunigter Strömungsgeschwindigkeit tauglich wird.

### Darlegung der Erfindung

Diese Aufgabe wird in Verbindung mit den Merkmalen des Oberbegriffs von Anspruch 1 dadurch gelöst, dass die Leitungszone eine Vielzahl vertiefter, offener Rinnen, die als Strömungskanäle ausgebildet sind, aufweist, welche durch mikroporöse Stege mit offenporigen Seitenwänden voneinander getrennt sind, wobei die Breite der Kanäle und der Stege sowie die Höhe der Stege und die Tiefe der Kanäle jeweils zwischen 5 Mikrometer und 100 Mikrometer liegen und wobei die Breite der Stege dem 0,5- bis 5fachen der Breite der Kanäle entspricht.

Bevorzugte Ausführungsformen der Erfindung sind Gegenstand der abhängigen Ansprüche.

Grundlage der Erfindung ist die Erkenntnis, dass die Laufgeschwindigkeit der Benetzungsfront einer Transportflüssigkeit in einer mikroporösen Transportschicht deutlich gesteigert werden kann, indem die Transportschicht eine Vielzahl von Strömungskanälen, die im Wesentlichen in Laufrichtung der Benetzungsfront verlaufen, aufweist, sofern die Seitenwände der Kanäle anders als bei bekannten Mikrofluidik-Kanälen nicht geschlossen, sondern offenporig sind, d.h. einen unverschlossenen Eingang in das offenporige Mikroporensystem der Transportschicht darstellen. Die genauen physikalischen Ursachen dieses Phänomens sind noch nicht geklärt. Es steht jedoch zu vermuten, dass die Benetzungscharakteristik der als relativ breite Kapillaren wirkenden Kanäle einerseits und der kapillarkrafterzeugenden Mikroporen der Transportschicht andererseits hier kapillarkraftverstärkend zusammenwirken.

Dies ermöglicht die Verwendung eines vergleichsweise kleinporigen Materials, was aufgrund seiner großen inneren Oberfläche für eine besonders dichte Anhaftung von selektiven Bindern als Voraussetzung eines scharfen Signals bei einer analytischen Testvorrichtung geeignet ist, wobei jedoch die nachteilige Bremswirkung auf die Strömungsgeschwindigkeit, die beim Stand der Technik mit der Kleinporigkeit der Transportschicht verbunden ist, durch die erfindungsgemäßen Kanäle behoben oder gar überkompensiert wird. Auf diese Weise lassen sich bessere Lösungen für das geschilderte Optimierungsdilemma zwischen Transportgeschwindigkeit und Signalschärfe finden.

Durch geeignete Wahl der Dimensionierung der Kanäle, insbesondere in ihrem Verhältnis zu den sie trennenden mikroporösen Stegen, lässt sich der erfindungsgemäß resultierende Beschleunigungseffekt in weiten Bereichen variieren. Spezielle, vorteilhafte Dimensionierungen sind daher Gegenstand der Erfindung.

Diese Abhängigkeit des Beschleunigungseffektes von der Relativdimensionierung hat eine wichtige praktische Bedeutung. Ist die Transportflüssigkeit nämlich besonders viskos oder enthält sie Feststoffe, wie beispielsweise Zellen oder Zellfragmente, müssen die Kanäle vergleichsweise breit gewählt werden, um eine Verstopfung zu verhindern. Gleichwohl kann über eine entsprechende Dimensionierung der Stege dennoch genau das gewünschte Ausmaß des erfindungsgemäßen Beschleunigungseffektes und damit die Laufzeit eingestellt werden.

Es ist vorgesehen, dass die Breite der Kanäle und der Stege sowie die Höhe der Stege und die Tiefe der Kanäle, d.h. insbesondere die Schichthöhe der Transportschicht, jeweils zwischen 5 Mikrometer und 100 Mikrometer liegen. Dabei ist es besonders vorteilhaft, wenn die Breite der Kanäle zwischen 10 und 50 Mikrometer liegt. Wie weiter unten anhand konkreter Versuchsmessungen noch detaillierter angegeben, hat sich die erfindungsgemäße Relativdimensionierung von Kanälen und Stegen, bei der die Breite der Stege dem 0,5- bis 5-fachen, insbesondere dem 0,9- bis 1,2-fachen der Breite der Kanäle entspricht, als besonders günstig erwiesen.

Wie oben bereits erwähnt, verlaufen die Kanäle im Wesentlichen in der Laufrichtung der Benetzungsfront der Transportflüssigkeit in der Transportschicht. "Im Wesentlichen" bedeutet in diesem Zusammenhang, dass die Kanäle nicht exakt entlang der Geraden zwischen Start- und Zielzone verlaufen müssen, sondern das vielmehr auch "Umwege" wie Schlangenlinien, Zickzack-Muster und ähnliches realisiert sein können, solange die wesentliche Richtungskomponente des Gesamtkanals von der Startzone zur Zielzone weist. Allerdings wird ein gerader Verlauf der Kanäle in den meisten Fällen, insbesondere herstellungstechnisch, günstig sein. Unabhängig von der konkreten Verlaufsform der Einzelkanäle hat es sich als günstig herausgestellt, wenn benachbarte Kanäle parallel zueinander verlaufen. Mit anderen Worten bleibt die Breite des Steges zwischen zwei benachbarten Kanälen bevorzugt über die Länge der Kanäle gleich. Wie erwähnt, hat die Relativdimensionierung von Stegen und Kanälen einen wesentlichen Einfluss auf das Ausmaß des erfindungsgemäßen Beschleunigungseffektes. Parallele Kanäle führen daher zu einer gleichmäßigen Ausprägung des Beschleunigungseffektes über die gesamte Kanallänge.

Obgleich es grundsätzlich denkbar ist, dass auch der Boden der Kanäle eine dünne Schicht eines mikroporösen Materials aufweist, hat es sich als günstig erwiesen, wenn der Boden der Kanäle durch den flüssigkeitsundurchlässigen Träger gebildet wird. Von einer mikroporösen Bodenschicht, die unter Berücksichtigung der im Hinblick auf die Einsparung von Transportflüssigkeit möglichst klein zu haltenden Gesamtschichtdicke der mikroporösen Transportschicht äußerst dünn sein müsste, dürfte kaum ein Beschleunigungseffekt zu erwarten sein, wie dies bei den mit dem ausgedehnten Schichtkörper der mikroporösen Transportschicht verbundenen Seitenwänden der Kanäle der Fall ist. Die durch die offenen Poren einer derartigen Bodenschicht realisierten Unebenheiten dürften eher eine Bremswirkung haben. Gleichwohl sind derartige Realisierungen der vorliegenden Erfindung nicht ausgeschlossen.

Die Flüssigkeitstransportvorrichtung der erfindungsgemäßen analytischen Testvorrichtung kann grundsätzlich beliebig komplex ausgestaltet sein. Insbesondere ist sie nicht auf einfache Strukturen mit nur einer Startzone und einer Zielzone mit nur einer dazwischenliegenden Transportzone beschränkt. Vielmehr können auch Varianten realisiert sein, bei denen auf dem Träger eine Mehrzahl voneinander getrennter Startzonen aufgebracht sind, die über eine Mehrzahl voneinander getrennter Leitungszonen mit einer gemeinsamen Zielzone verbunden sind. Umgekehrt ist es auch möglich, dass auf dem Träger eine Mehrzahl voneinander getrennter Zielzonen aufgebracht sind, die über eine Mehrzahl voneinander getrennter Leitungszonen mit einer gemeinsamen Startzone verbunden sind. Dabei ist es nicht erforderlich, dass die jeweiligen Leitungszonen parallel zueinander verlaufen. Vielmehr können sie auch unter einem von Null Grad verschiedenen Winkel zueinander verlaufen. Auch Kreuzungen von Leitungszonen, in den sich bislang getrennte Anteile der Transportflüssigkeit oder unterschiedlicher Transportflüssigkeiten mischen, sind denkbar. Schließlich ist es im Rahmen der vorliegenden Erfindung auch nicht erforderlich, dass jeweils einem Paar von Start- und Zielzone genau eine Leitungszone zugeordnet ist. Vielmehr kann ein solches Paar von Start- und Zielzone über eine Mehrzahl von voneinander getrennten Leitungszonen miteinander verbunden sein. Beispielsweise ist denkbar, dass die Transportflüssigkeit von einer Startzone der Zielzone auf unterschiedlichen langen Wegen oder auf Wegen mit unterschiedlichen Kanalbreiten zugeführt wird.

Weitere Merkmale und Vorteile der Erfindung ergeben sich aus der nachfolgenden ausführlichen Beschreibung und den Zeichnungen.

### Kurzbeschreibung der Zeichnungen

In den Zeichnungen zeigen:
- Figur 1:: eine schematische Draufsicht auf eine Ausführungsform einer erfindungsgemäßen Testvorrichtung,
- Figur 2:: eine schematische Ausrissdarstellung der Schnittansicht entlang der Schnittlinie II-II in Figur 1,
- Figur 3:: eine rasterelektronenmikroskopische Aufnahme einer mit einem Nd:YVO4-Festkörperlaser erzeugbaren erfindungsgemäßen Kanalstruktur,
- Figur 4:: eine rasterelektronenmikroskopische Aufnahme einer mit einem CO₂-Laser im Infrarotbereich erzeugbaren Kanalstruktur als Vergleichsbeispiel,
- Figur 5:: eine Ausführungsform einer erfindungsgemäßen Testvorrichtung und
- Figur 6:: eine weitere Ausführungsform einer erfindungsgemäßen Testvorrichtung.

Ausführliche Beschreibung bevorzugter Ausführungsformen

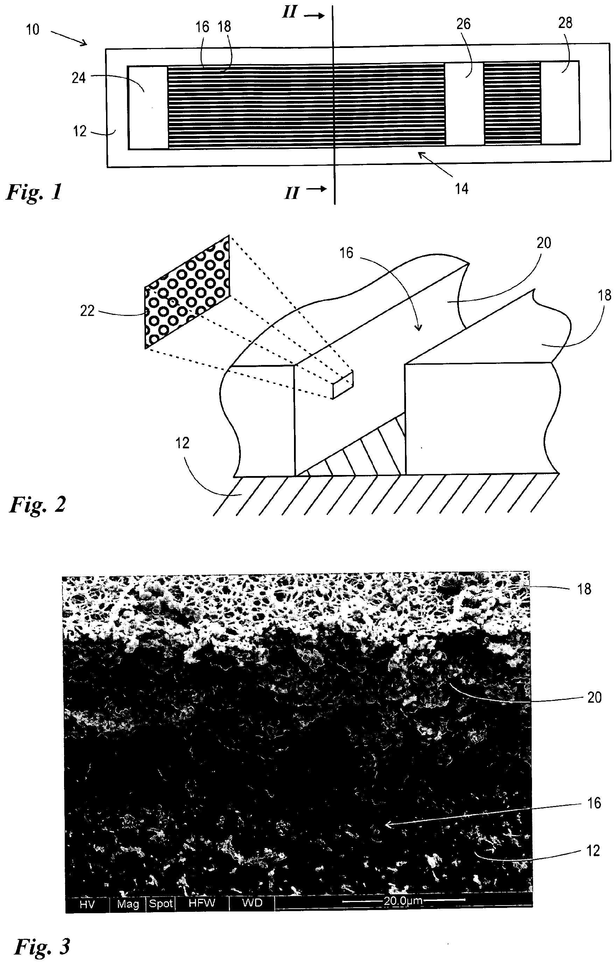

Figur 1 zeigt in stark schematisierter Darstellung eine Draufsicht auf eine erfindungsgemäße Testvorrichtung. Die Figuren 2 und 3 zeigen jeweils Details der Vorrichtung von Figur 1. Die Figuren 1 bis 3 sollen nachfolgend gemeinsam diskutiert werden.

Gleiche Bezugszeichen bezeichnen gleiche oder entsprechende Elemente in allen Figuren.

Die Testvorrichtung 10 gemäß Figur 1 entspricht in ihrem grundsätzlichen Aufbau bekannten Lateral-Flow-Assays. Basis der Vorrichtung ist ein Träger 12, der beispielsweise aus Glas, insbesondere Borosilikatglas, Kunststoff oder einem anderen flüssigkeitsundurchlässigen Material gefertigt ist, wobei in der Regel auf eine möglichst geringe Reaktivität mit Bestandteilen der vorgesehenen Proben und Reagenzien zu achten sein wird.

Häufig wird es günstig sein, eine Oberflächenfunktionalisierung, z.B. eine Aminosilanbeschichtung des Trägers 12, vorzunehmen. Die Oberflächenmodifizierung hat den Vorteil, dass eine nachfolgend auf dem Träger aufgebrachte mikroporöse Transportschicht besser haftet. Als mikroporöse Trägerschicht kann beispielsweise Zellulosenitrat, Polyamid, Polysulfon, PVDF, poröse Keramiken und anderes verwendet werden.

Bei einer bevorzugten Ausführungsform kann eine fertige mikroporöse Membran als mikroporöse Trägerschicht auf den optional wie vorstehend beschrieben modifizierten Träger 12 aufgeklebt/laminiert werden.

Die mikroporöse Trägerschicht kann auch aus mehreren stofflich unterschiedlichen Schichten der vorgenannten Materialien oder aus stofflich identischen Schichten mit unterschiedlicher Porengrößenverteilung bestehen, wobei diese Mehrschichten-Strukturen durch dem Fachmann bekannte "Cocasting"-Verfahren auf den Träger aufgebracht werden können.

Beispielhaft sei das Aufbringen einer Gießlösung aus einem Polymerblend von handelsüblichem Zellulosenitrat (5% bis 10%) und ggf. Zelluloseazetat (kleiner 2%) in einem Lösungsmittelgemisch aus Methylazetat (40%-60%), Alkoholen (30%-50%) und Wasser angegeben. Die Gießlösung kann zudem übliche Netzmittel zur Gewährleistung einer sicheren Benetzung enthalten, beispielsweise SDBS (Sodium Dodecyl Benzene Sulfonate) oder SDS (Sodium Dodecyl Sulfate) jeweils in einem Gewichtsanteil unter 0,5%.

Beim Trocknen der auf dem Träger 12 aufgebrachten Gießlösungsschicht entsteht bei Verdunstung der überwiegenden Bestandteile des Lösungsmittelgemisches unter Phaseninversion eine poröse Transportschicht 14. Die resultierenden Dicken der Transportschicht 14 werden durch die Beschichtungsdicke der Gießlösung gesteuert. Schichtdicken von ca. 100 bis 500 Mikrometer Nassdicke führen hierbei zu Trockendicken von 10 bis 100 Mikrometer. Nach Trocknung der Transportschicht 14 wird diese strukturiert, indem die mikroporöse Schicht bereichsweise entfernt wird, sodass eine Vielzahl von Kanälen 16 entsteht, zwischen denen jeweils Stege 18 aus mikroporösem Material stehen bleiben. Dabei ist, wie in Figur 2 durch den vergrößerten Ausschnitt 22 schematisch angedeutet und in Figur 3 in der rasterelektronenmikroskopischen Aufnahme deutlich zu erkennen ist, darauf zu achten, dass die Materialporen an der Oberfläche der Kanalwände 20, d.h. an den Stegseiten nicht verschlossen werden, sondern offen bleiben, sodass Flüssigkeit aus dem Kanal in das mikroporöse Stegmaterial eindringen kann.

Dies gelingt beispielsweise durch Laserstrukturierung mit auf das jeweilige Transportschichtmaterial abgestimmten Lasersystemen. Für die oben beispielhaft im Detail erläuterten Zellulosenitratschichten hat sich ein Nd:YVO4-Festkörperlaser mit Picosekunden-Pulsen, insbesondere bei einer Wellenlänge von 532 Nanometern und einer Pulslänge von 12 Picosekunden, einer Pulsenergie von 10 Mikrojoule und einer Pulsfrequenz von 10 Kilohertz, dessen Strahl mittels einer 100 Millimeter F-Theta-Optik und einem Vorschub von 25 Millimetern pro Sekunde auf die Transportschicht fokussiert wurde, als geeignet erwiesen. Als ungeeignet hat sich hingegen ein CO₂-Laser im Infrarotbereich erwiesen, der zu einer Verschmelzung und somit zum Verschluss der Kanalwandporen geführt hat.

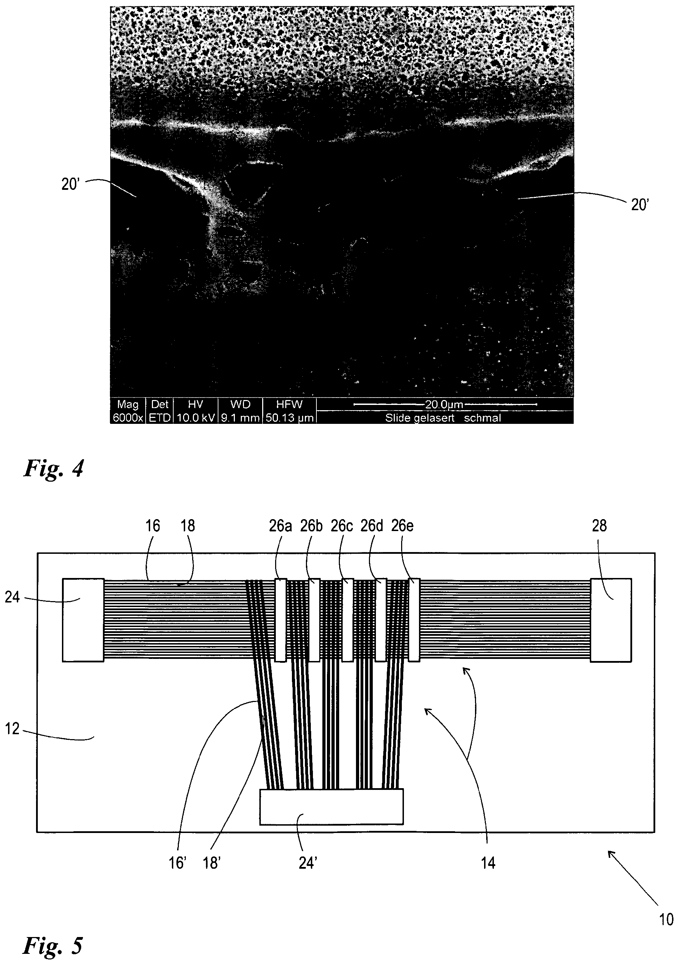

Nach Figur 4 resultiert bei der Verwendung eines CO₂-Lasers im Infrarotbereich eine Kanalstruktur, die sich von der erfindungsgemäßen Kanalstruktur der Figur 3 dadurch unterscheidet, dass sie keine offenporigen Kanalwände 20 aufweist, sondern dass die Kanalwände 20' eine weitgehend geschlossene Struktur mit inhomogen über die Kanalwände 20' verteilten Durchbrüchen zeigen.

Alternativ zur Laserstrukturierung sind selbstverständlich auch mechanische oder chemische, insbesondere Ätzverfahren einsetzbar. Zur geeigneten Dimensionierung der Kanäle 16 bzw. der Stege 18 sollen weiter unten noch detaillierte Beispiele angegeben werden.

In Figur 1 sind weiter eine Startzone 24 und zwei Zielzonen 26, 28 eingezeichnet. Ihre Darstellung als Rechtecke mit homogener Oberfläche ist rein schematisch zu verstehen. Insbesondere die erste Zielzone 26 kann optisch ununterscheidbar von der strukturierten Transportschicht 14 gestaltet sein. Bevorzugt wird sie lediglich dadurch definiert, dass das poröse Material der Stege in der ersten Zielzone 26 mit immobilisierten, selektiven Bindern belegt ist, die in der Lage sind, zugeführten, markierten Analyten in der Zielzone 26 zu fixieren. Die Startzone 24 und die zweite Zielzone 28 umfassen jedoch bevorzugt zusätzliche Reservoirkissen, beispielsweise in Form von Glasfaservliesen, die in Flüssigkeit leitender Weise mit der Transportschicht 14 verbunden sind. Auf diese Weise kann in der Startzone ein größeres Volumen Probenflüssigkeit hinterlegt werden, welche kontinuierlich als Transportflüssigkeit in die Transportschicht abgegeben und durch die weiter oben beschriebene, vermutete Wechselwirkung zwischen den Kapillaritäten der porösen Stege und der Kanäle in Richtung der Zielzonen 26, 28 strömt. Das Reservoirkissen der zweiten Zielzone 28 dient der Aufrechterhaltung des Transportstroms auch dann, wenn die Benetzungsfront der Transportflüssigkeit das stromabwärts gelegene Ende der Transportschicht 14 erreicht hat.

Der erfindungsgemäße Flüssigkeitstransport-Beschleunigungseffekt soll nachfolgend an ausgewählten Beispielen belegt werden. Hierzu wurden unterschiedlich dicke Zellulosenitratschichten 14 in der oben im Detail geschilderten Weise auf Borosilikat-Träger 12 aufgebracht und mit dem oben als bevorzugt geschilderten Lasersystem in unterschiedlicher Weise, d.h. mit unterschiedlichen Kanal- und Stegbreiten, strukturiert. Die Kanaltiefe bzw. Steghöhe entsprach dabei jeweils der Schichtdicke, d.h. der Kanalboden wurde jeweils von dem Material des Trägers 12 gebildet. Nach Auftrag einer wässrigen Lösung in der Startzone 24 wurde diejenige Zeit gemessen, die die Benetzungsfront benötigte, um eine Strecke von 40 Millimetern zurückzulegen. Zum Vergleich wurde jeweils eine Messung an einer nicht strukturierten, im Übrigen jedoch identischen Flüssigkeitstransportvorrichtung durchgeführt. Die nachfolgenden Tabellen geben die jeweiligen Ergebnisse wieder:

**Tabelle 1: Wandergeschwindigkeit der Benetzungsfront in sec/40 mm, Schichtdicke der Transportschicht: 70 Mikrometer Referenz (ohne Struktur): 250 sec/40 mm**

| Kanalbreite [um] →: Stegbreite [µm]: ↓ | 20 | 50 |
|---|---|---|
| 20 | 8 | - |
| 50 | - | 7 |
| 100 | 21 | - |
| 250 | - | 28 |

Es ist eine deutliche Beschleunigung der Wandergeschwindigkeit gegenüber der unstrukturierten Referenz erkennbar. Bei gleicher Dimensionierung von Kanälen 16 und Stegen 18 ist der erfindungsgemäße Beschleunigungseffekt deutlich stärker ausgeprägt als bei breiterer Dimensionierung der Stege 18 im Vergleich zu den Kanälen 16.

**Tabelle 2: Wandergeschwindigkeit der Benetzungsfront in sec/40 mm, Schichtdicke der Transportschicht: 35 Mikrometer Referenz (ohne Struktur): 570 sec/40 mm**

| Kanalbreite [µm] →: Stegbreite [µm]: ↓ | 20 | 50 |
|---|---|---|
| 20 | 12 | - |
| 50 | - | 15 |
| 60 | 22 | - |
| 100 | 36 | - |
| 150 | - | 31 |
| 250 | - | 54 |

Es sind die gleichen Effekte und Tendenzen zu erkennen wie in Tabelle 1, wobei insgesamt die reduzierte Schichtdicke zu einer Verlangsamung der Strömungsgeschwindigkeit führt.

**Tabelle 3: Wandergeschwindigkeit der Benetzungsfront in sec/40 mm, Schichtdicke der Transportschicht: 10 Mikrometer Referenz (ohne Struktur): >1800 sec/40 mm**

| Kanalbreite [µm] →: Stegbreite [µm] : ↓ | 20 | 50 |
|---|---|---|
| 20 | 58 | - |
| 50 | - | 137 |
| 60 | 85 | - |
| 100 | 96 | - |
| 150 | - | 245 |
| 250 | - | 252 |

Innerhalb der jeweils selben Kanalbreite sind wieder die gleichen Tendenzen wie in den Tabellen 1 und 2 zu erkennen. Allerdings ist bei der hier realisierten, besonderes geringen Schichtdicke der Transportschicht der Einfluss des Absolutwertes der Kanalbreite deutlich stärker als bei den zuvor betrachteten, größeren Schichtdicken. Die Referenzmessung wurde nach 30 min abgebrochen, da die Wandergeschwindigkeit der Benetzungsfront so gering war, dass hier kaum sinnvolle Anwendungen denkbar sind. Andererseits sind jedoch geringe Schichtdicken gerade für Anwendungen mit geringen Flüssigkeitsmengen interessant, sodass hier die vorliegende Erfindung besonders wertvoll ist.

Figur 5 zeigt eine weitere Ausführungsform einer erfindungsgemäßen Testvorrichtung in schematischer Darstellung. Es handelt sich hierbei um eine bidirektionale Ausführungsform, die im Gegensatz zu der unidirektionalen Ausführungsform von Figur 1 zwei im Wesentlichen senkrecht aufeinander stehende Hauptflussrichtungen von Transportflüssigkeit zeigt. Zusätzlich zu der Startzone 24 ist eine weitere Startzone 24' auf dem Träger 12 vorgesehen. Die Transportschicht 14 ist in einem ersten Bereich vergleichbar mit der Ausführungsform von Figur 1 strukturiert, d.h. mit geraden, parallelen Kanälen 16, die sich von der Startzone 24 zur zweiten Zielzone 28 erstrecken und dabei eine in fünf Teilzielzonen 26a-e aufgeteilte erste Zielzone passieren. In einem zweiten Bereich ist die Transportschicht 14 in jeweils wiederum parallel zueinander verlaufende Kanäle 16' umfassende Gruppen von Kanälen 16' strukturiert, die sich von der zusätzlichen Startzone 24' bis in die unmittelbare Nachbarschaft der Teilzielzonen 26a-e erstrecken. Wie schematisch dargestellt, sind bei der gezeigten Ausführungsform die zweiten Kanäle 16' breiter gestaltet als die ersten Kanäle 16, sodass in diesem Bereich eine Transportstruktur realisiert ist, die toleranter gegen in der Transportflüssigkeit mitgeführte Feststoffe ist. Zum Betrieb dieser Vorrichtung wird in der Startzone 24 eine erste Flüssigkeit aufgebracht, die bestimmte Reagenzien enthält oder aus der Startzone 24 herauswäscht. Über die Kanäle 16 wird ein Hauptstrom zur zweiten Zielzone 28 realisiert. Gleichzeitig, vorher oder später wird in der zusätzlichen Startzone 24' eine zweite Flüssigkeit aufgebracht, die beispielsweise eine Probenflüssigkeit sein kann, welche Feststoffe wie Zellen oder Zellfragmente enthält. Diese Flüssigkeit gelangt über die Kanäle 16' unmittelbar in die Nähe je einer der ersten Teilzielzonen 26a-e und zwar im Hinblick auf die vorgenannte Hauptstromrichtung jeweils stromaufwärts von diesen. Die schwer transportierbare Flüssigkeit wird somit über speziell dimensionierte Kanäle 16' zu den ersten Teilzielzonen 26a-e befördert, wo sie sich mit der über die schmaleren Kanäle 16 beigeführten Reagenzlösung mischen kann. Diese Mischung kann beispielsweise zu einer durch eine Immuno-Reaktion erfolgte Markierung eines Analyten führen. Eine Fixierung des markierten Analyten kann mit entsprechenden, immobilisierten Bindern in den ersten Teilzielzonen 26a-e in bekannter Weise erfolgen, was trotz der scheinbaren Unterdimensionierung der Kanäle 16 für die schwer transportable Probenflüssigkeit aufgrund der sehr geringen, zu überbrückenden Strecke zügig möglich ist.

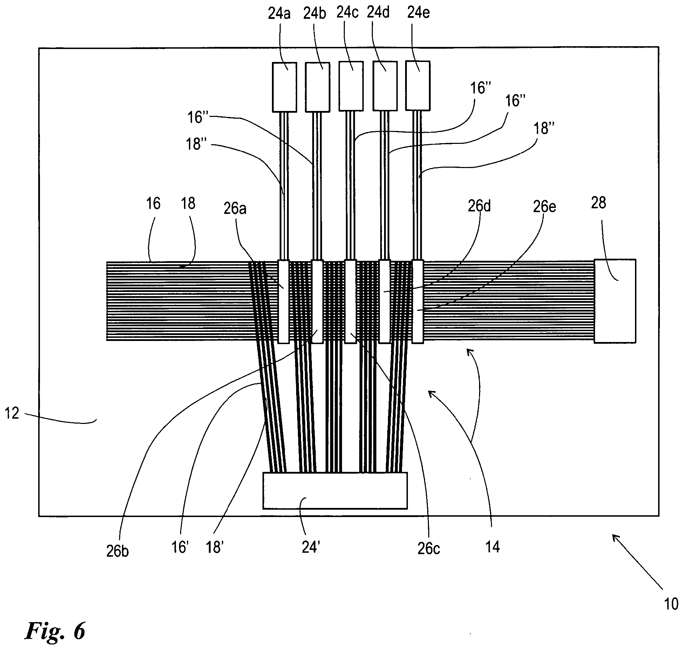

Bei der gezeigten Ausführungsform kreuzen sich die Kanäle 16 und 16' in derselben Ebene. Dies hat den Vorteil, dass die gesamte Kanal/Steg-Struktur der Vorrichtung von Figur 4 aus einer gemeinsamen Transportschicht 14, mit der Träger 12 ursprünglich belegt war, herausgearbeitet werden kann. Selbstverständlich sind im Rahmen der vorliegenden Erfindung jedoch auch Ausführungsformen möglich, bei denen sich unterschiedliche Kanalgruppen nicht kreuzen, sondern nur aneinander anstoßen. Auch sind Varianten denkbar, bei denen unterschiedliche Transportschichtebenen realisiert sind. Eine weitere in Figur 6 dargestellte Variante der Ausführungsform gemäß Figur 5 weist statt der einen Startzone 24 im linken Bereich des Trägers 12 aus Figur 5 eine Mehrzahl von Startzonen 24a-e auf, die über mehrere Leitungswege, die Gruppen von Kanälen 16'' bzw. Stegen 18'' umfassen, mit den Teilzielzonen 26a-e in kommunizierender Verbindung stehen. Alternativ kann die Mehrzahl von Startzonen 26a-e auch zu einer einzigen Startzone fusioniert sein, welche über die vorgenannten Leitungswege parallel mit den Teilzielzonen 26a-e verbunden ist.

### Bezugszeichenliste

- 10: Testvorrichtung
- 12: Träger
- 14: Transportschicht
- 16: Kanal
- 16', 16": weitere Kanäle
- 18: Steg
- 18', 18": weitere Stege
- 20, 20': Stegwand/Kanalwand
- 22: Vergrößerungsfenster von 20
- 24, 24a-e: Startzone
- 24': zusätzliche Startzone
- 26: erste Zielzone
- 26a-e: erste Teilzielzonen
- 28: zweite Zielzone

## Patentansprüche

1. Analytische Testvorrichtung, umfassend eine Flüssigkeitstransportvorrichtung mit einem flüssigkeitsdichten Träger (12), darauf aufgebracht eine Startzone (24; 24') zur Aufbringung von zu transportierender Transportflüssigkeit und eine Zielzone (26, 28; 26a-e), in die die Transportflüssigkeit zu transportieren ist, sowie eine sich zwischen der Startzone (24, 24') und der Zielzone (26, 28; 26a-e) erstreckende Leitungszone, die eine mikroporöse Transportschicht (14) umfasst, in der die Transportflüssigkeit kapillarkraftvermittelt von der Startzone (24; 24') zur Zielzone (26, 28; 26a-e) strömt, wobei in wenigstens einer Zielzone (26, 28; 26a-e) ein selektiver Binder dauerhaft immobilisiert ist, der mit einem mit der Transportflüssigkeit aus der Startzone (24; 24', 24a-e)in die Zielzone (26, 28; 26a-e) transportierten, markierten Analyten selektiv bindungsfähig ist,
**dadurch gekennzeichnet,**
**dass** die Leitungszone eine Vielzahl vertiefter, offener Rinnen (16, 16'), die als Strömungskanäle ausgebildet sind, aufweist, welche durch mikroporöse Stege (18; 18') mit offenporigen Seitenwänden (20; 20') voneinander getrennt sind, wobei die Breite der Kanäle (16; 16') und der Stege (18; 18') sowie die Höhe der Stege (18; 18') und die Tiefe der Kanäle (16; 16') jeweils zwischen 5 Mikrometer und 100 Mikrometer liegen und wobei die Breite der Stege (18; 18') dem 0,5- bis 5fachen der Breite der Kanäle (16; 16') entspricht.

2. Analytische Testvorrichtung nach Anspruch 1,
**dadurch gekennzeichnet,**
**dass** in wenigstens einer Startzone (24; 24') ein im trockenen Zustand immobilisierter und im feuchten Zustand beweglicher, markierter, selektiver Binder vorliegt, der mit einem in der Transportflüssigkeit enthaltenen Analyten selektiv bindungsfähig ist.

3. Analytische Testvorrichtung nach einem der vorangehenden Ansprüche,
**dadurch gekennzeichnet,**
**dass** die Breite der Stege (18; 18') dem 0,9- bis 1,2fachen der Breite der Kanäle (16; 16') entspricht.

4. Analytische Testvorrichtung nach Anspruch 3,
**dadurch gekennzeichnet, dass** die Breite der Kanäle (16; 16') zwischen 10 und 50 Mikrometer liegt.

5. Analytische Testvorrichtung nach einem der vorangehenden Ansprüche,
**dadurch gekennzeichnet,**
**dass** die Kanäle (16; 16') parallel zueinander verlaufen.

6. Analytische Testvorrichtung nach einem der vorangehenden Ansprüche,
**dadurch gekennzeichnet,**
**dass** der Boden der Kanäle (16; 16') durch den flüssigkeitsundurchlässigen Träger (12) gebildet wird.

7. Analytische Testvorrichtung nach einem der vorangehenden Ansprüche,
**dadurch gekennzeichnet,**
**dass** auf dem Träger eine Mehrzahl voneinander getrennter Startzonen aufgebracht sind, die über eine Mehrzahl voneinander getrennter Leitungszonen mit einer gemeinsamen Zielzone verbunden sind.

8. Analytische Testvorrichtung nach einem der vorangehenden Ansprüche,
**dadurch gekennzeichnet,**
**dass** auf dem Träger (12) eine Mehrzahl voneinander getrennter Zielzonen (26, 28) aufgebracht sind, die über eine Mehrzahl voneinander getrennter Leitungszonen mit einer gemeinsamen Startzone (24) verbunden sind.

9. Analytische Testvorrichtung nach einem der vorangehenden Ansprüche,
**dadurch gekennzeichnet,**
**dass** die Startzone (24') und die Zielzone (28) über eine Mehrzahl voneinander getrennter Leitungszonen miteinander verbunden sind.

## Claims

1. Analytical testing device comprising a fluid transport device with a fluid-impermeable support (12), upon which support a start zone (24; 24') is arranged for applying transport fluid to be transported and a target zone (26, 28; 26a-e) into which the transport fluid is to be transported, as well as a conduction zone extending between the start zone (24, 24') and the target zone (26, 28; 26a-e) that comprises a microporous transport layer (14) in which the transport fluid flows by capillary action from the start zone (24; 24') to the target zone (26, 28; 26a-e), wherein a selective binder is permanently immobilized in at least one target zone (26, 28; 26a-e), which binder is capable of selectively binding with marked analytes transported by the transport fluid from the start zone (24; 24', 24a-e) to the end zone (26, 28; 26a-e),
**characterized in that**
the conduction zone has a plurality of deepened, open grooves (16, 16') designed as flow channels that are separated from one another by microporous dividers (18; 18') with open-pore side walls (20; 20'), wherein the width of the channels (16; 16') and the dividers (18; 18') as well as the height of the dividers (18; 18') and the depth of the channels (16; 16') are respectively between 5 microns and 100 microns and wherein the width of the dividers (18; 18') corresponds to 0.5 to 5 times the width of the channels (16; 16').

2. Analytical testing device according to claim 1,
**characterized in that**
in at least one start zone (24; 24') a binder is present that in the dry state is immobilized and in the wet state is mobile, marked and selective, and that is selectively bondable with an analyte contained in the transport fluid.

3. Analytical testing device according to any one of the preceding claims,
**characterized in that**
the width of the dividers (18; 18') corresponds to 0.9 to 1.2 times the width of the channels (16; 16').

4. Analytical testing device according to claim 3, **characterized in that** the width of the channels (16; 16') lies between 10 and 50 microns.

5. Analytical testing device according to any one of the preceding claims,
**characterized in that**
the channels (16; 16') run parallel to one another.

6. Analytical testing device according to any one of the preceding claims,
**characterized in that**
the base of the channels (16; 16') is formed by the fluid-impermeable support (12).

7. Analytical testing device according to any one of the preceding claims,
**characterized in that**
a plurality of start zones separated from one another are applied on the support, which start zones are connected to a common target zone by a plurality of conduction zones that are separated from one another.

8. Analytical testing device according to any one of the preceding claims,
**characterized in that**
a plurality of target zones (26, 28) separated from one another are applied on the support (12), which target zones are connected to a common start zone (24) by a plurality of conduction zones that are separated from one another.

9. Analytical testing device according to any one of the preceding claims,
**characterized in that**
the start zone (24') and the target zone (28) are connected to one another via a plurality of conduction zones that are separated from one another.

## Revendications

1. Dispositif de test analytique comprenant un dispositif de transport de liquides doté d'un support (12) étanche aux liquides, une zone de départ (24 ; 24') placée sur celui-ci, destinée à l'application du liquide de transport à transporter, et une zone cible (26, 28 ; 26a-e) destinée à recevoir le liquide de transport, ainsi qu'une zone de conduite s'étendant entre la zone de départ (24, 24') et la zone cible (26, 28 ; 26a-e) et comprenant une couche de transport (14) où circule le liquide de transport par capillarité entre la zone de départ (24 ; 24') et la zone cible (26, 28 ; 26a-e), sachant qu'au moins dans une zone cible (26, 28 ; 26a-e) un liant sélectif est immobilisé de façon permanente, ledit liant pouvant s'attacher de manière sélective à un analyte marqué, transporté avec le liquide de transport de la zone de départ (24 ; 24', 24a-e) vers la zone cible (26, 28 ; 26a-e),
**caractérisé en ce**
**que** la zone de conduite présente une pluralité de goulottes (16, 16') profondes et ouvertes, réalisées en forme de canaux d'écoulement, qui sont séparées les unes des autres par des entretoises (18 ; 18') microporeuses aux parois latérales (20 ; 20') poreuses, sachant que la largeur des canaux (16 ; 16') et des entretoises (18 ; 18') ainsi que la hauteur des entretoises (18 ; 18') et la profondeur des canaux (16 ; 16') mesurent respectivement entre 5 et 100 micromètres et que la largeur des entretoises (18 ; 18') correspond à 0,5 à 5 fois la largeur des canaux (16 ; 16').

2. Dispositif de test analytique selon la revendication 1,
**caractérisé en ce**
**qu'**un liant sélectif marqué, immobilisé à l'état sec et mobile à l'état humide, se trouve au moins dans une zone de départ (24 ; 24') et peut s'attacher de manière sélective à un analyte contenu dans le liquide de transport.

3. Dispositif de test analytique selon l'une des revendications précédentes,
**caractérisé en ce**
**que** la largeur des entretoises (18 ; 18') correspond à 0,9 à 1,2 fois la largeur des canaux (16 ; 16').

4. Dispositif de test analytique selon la revendication 3,
**caractérisé en ce que** la largeur des canaux (16; 16') mesure entre 10 et 50 micromètres.

5. Dispositif de test analytique selon l'une des revendications précédentes,
**caractérisé en ce**
**que** les canaux (16 ; 16') sont parallèles entre eux.

6. Dispositif de test analytique selon l'une des revendications précédentes,
**caractérisé en ce**
**que** le fond des canaux (16 ; 16') est constitué par le support (12) étanche aux liquides.

7. Dispositif de test analytique selon l'une des revendications précédentes,
**caractérisé en ce**
**que** le support comporte une pluralité de zones de départ séparées les unes des autres et reliées avec une zone cible commune par une pluralité de zones de conduite séparées les unes des autres.

8. Dispositif de test analytique selon l'une des revendications précédentes,
**caractérisé en ce**
**que** le support (12) comporte une pluralité de zones cibles (26, 28) séparées les unes des autres et reliées avec une zone de départ (24) commune par une pluralité de zones de conduite séparées les unes des autres.

9. Dispositif de test analytique selon l'une des revendications précédentes,
**caractérisé en ce**
**que** la zone de départ (24') et la zone cible (28) sont reliées l'une à l'autre par une pluralité de zones de conduite séparées les unes des autres.
